# EUROPEAN PATENT SPECIFICATION

(11) **EP 4 329 665 B1**
(45) Date of publication and mention of the grant of the patent: **20.05.2026**
(21) Application number: 22726169.0
(22) Date of filing: 12.04.2022
(51) Int. Cl.: A61C 8/00, A61B 17/16, A61C 1/08, A61B 90/00

(54) **COMPONENT KIT FOR DENTAL IMPLANTOLOGY**
KOMPONENTENSATZ FÜR DIE ZAHNIMPLANTOLOGIE
KIT DE COMPOSANTS POUR IMPLANTOLOGIE DENTAIRE

(30) Priority: 27.04.2021 IT 202100010646
(43) Date of publication of application: 06.03.2024
(73) Proprietor: Sweden & Martina SpA, 35020 Due Carrare (PD) (IT)
(72) Inventor: MARTINA, Alberto, 35020 Due Carrare (PD) (IT)
(74) Representative: Rocchetto, Elena
(86) International application number: PCT/IB2022/053411
(87) International publication number: WO 2022/229768

(56) References cited:
- WO-A1-2008/088105
- WO-A1-2018/071863
- US-A1- 2007 099 150

## Description

This patent relates to the components used in implantology surgical procedures and in particular, concerns a new bur equipped with a system for locking the stop element.

A document in the art is exemplified by WO 2008/088105 A1.

In modern implantology, the prior art includes the use of surgical burs in combination with so-called "drill stop" elements, which are mainly used for safety purposes if the area to be prepared for the subsequent insertion of the implant has a reduced vertical length or in any case very similar to the length of the implant to be inserted. Therefore, in order to prevent any damage to the mandibular nerves, it is absolutely necessary to limit the maximum drilling depth.

Such stop elements of the prior art have different lengths and diameters depending on the length and diameter of the implant to be inserted.

Said stop elements generally consist of a cylindrical tubular body in which the bur is inserted, so that the tip of the bur comes out of the cylindrical body for a given length. The stop elements of the prior art are therefore made of a cylindrical tubular body and a plurality of teeth arranged on one end of the cylindrical tubular body and placed parallel to the longitudinal axis of the cylindrical body.

Said teeth enable the coupling with the bur, which in turn is equipped with a protruding cylindrical portion, called "hub", in which the teeth of the stop element slide until reaching a determined final position, with or without interlocking or "clicking" that allows the operator to understand whether the proper engagement occurred or not. Stop elements of the prior art have some drawbacks.
1. Due to repeated use, teeth can deform, affecting the engaging capacity, reducing it, or even making the engagement impossible. The precise calibration of the teeth is very complex and the only way to obtain satisfactory results is to find the right balance between the number and size of the teeth, which can vary from 2 to 8 or more depending on the size.
2. This engagement system, especially when the "click" is expected to verify the proper engagement, involves very tight tolerances and a complex production process, including quality control, which exacerbates the final cost of the instruments.
3. During bone drilling, the stop element rests on the bone crest and inevitably tends to lock while the bur continues to turn at high speed because it is connected to the micro motor. This relative motion between the two components tends to release the stop element from the bur with the risk that, when removing the drill from the oral cavity, the stop element may drop and even be swallowed by the patient.
4. In addition, these surgical instruments are closely related to the implant diameter and length and therefore need to be marked with a color code. To do this, it is necessary to provide an area to insert a groove to be filled with a special colored resin on both components, that is, bur and stop element. Frequently this involves having to further lengthen the burs, which are often already too long, to have the space necessary to engrave the groove which again, is a costly process, since it involves complex operations and the procurement of certified colored resins. All this leads to high final production cost of the components.

To address these and other drawbacks, a new bur was designed and constructed with a stop element locking system.

The main task of this invention is to replace the teeth of the stop elements of the prior art with a new coupling system between the bur and the stop element able to transfer the "elastic" retaining function from the stop element, currently carried out by the teeth, to the bur.

The invention is defined by independent claim 1. Further embodiments are defined in dependent claims 2 to 8.

In a first example, the bur comprises a retaining component to be pre-assembled to the bur, wherein said retention component is a ring, preferably made of titanium, whether completely or partially, to be positioned in a circular groove made on the body of the bur itself.

The thickness of the ring is such as to protrude in part from the groove, which will therefore preferably have a smaller depth than the ring.

The stop element, on the other hand, has a cylindrical tubular shape with no teeth. This configuration has numerous advantages:
1. The drawback related to the wear/deformation of the teeth, which are not present on the stop element, is eliminated.
2. Apart from said ring, both the bur and the stop element can be produced with greater tolerances which translates into higher productivity and repeatability as well as lower production costs compared to the prior art.
3. The interlocking between the bur and the stop element takes place thanks to said ring that can rotate with respect to the bur inside the groove made on the bur itself. Thanks to this feature, in the case in which the stop element rests on the bone ridge, the bur can freely rotate with minimal friction with respect to the stop element and consequently the detachment will not occur as previously described.
4. Both said ring and the stop element can be made of the same material, for example and preferably of titanium. In this way it would be possible to take advantage of the anodic coloring process to color them both completely, thus without having to insert a colored ring as happens in the previously described expensive solutions of the prior art.

In a second example, the retaining component pre-assembled on the bur comprises at least one whole ball or part of a ball, or a body in general, inserted and confined within a seat in the bur body by at least one elastic means that constrains said ball in a position at least partially protruding from said seat, further counteracting the movement of the ball inwards.

Thus the stop element, inserted on the bur, is constrained in the proper position by the negative allowance created by the action of said at least one ball.

In a third possible example, the retaining component is integral with the bur body, and comprises at least one tab or elastically deformable element or projection, which protrudes from the bur body in a position such as to be at least partially interposed between the bur body and the stop element properly mounted thus creating the negative allowance.

In conclusion, according to the present invention, the new implantology bur comprises at least one retaining component for the stop element, wherein said retaining component is not part of the stop element, as is the case in the prior art, but is constrained to the bur itself.

Thanks to this feature, the stop element is free of retaining teeth, but comprises a cylindrical tubular body.

The characteristics of the new bur will be better clarified by the following description with reference to the drawings, attached by way of a non-limiting example.

Figure 1 shows an embodiment of the aforementioned prior art, where the bur (100) comprises a protruding cylindrical portion or "hub" (101) for the coupling of the teeth (210) of the stop element (200). The colored rings (110, 220) that identify the size of the bur (100) and the corresponding stop element (200) are also shown in Figure 1.

Figure 2 shows a first embodiment of the invention, where a bur (100), a retaining ring (301) and a stop element (200) are visible.

Figure 2a shows the stop element (200) fitted on the bur (100) with the retaining ring (301) between them.

Figure 2b shows an alternative embodiment, where the retaining ring (301') has a circular outer surface (311'), while it has a non-circular inner surface (310').

Figure 3 shows a second embodiment of the invention, where a stop element (200) and a bur (100') with retaining balls (302) are visible.

Figure 3a shows a section of the stop element (200) and the bur (100') of Figure 3, while Figure 3b shows the section of the bur (100') with the stop element (200) during its insertion, where the point of negative allowance between the stop element (200) and the retaining balls (302) is visible.

Figure 3c shows a detail of Figure 3b.

Figure 4 shows a third embodiment of the invention, where a stop element (200) and a bur (100") with deformable elastic retaining elements or projections (303) are shown.

Figure 4a shows a section of the stop element (200) and the bur (100") of Figure 4, while Figure 4b shows the section of the bur (100") with the stop element (200) during its insertion, where the point of negative allowance between the stop element (200) and the deformable retaining elements (303) is visible.

Figure 4c shows a detail of Figure 4b.

The invention is an implantology bur (100) comprising a cylindrical portion (101) for positioning at least one stop element (200) used for adjusting the drilling depth.

The bur (100) comprises at least one retaining component (300) suited to create a negative allowance with said stop element (200) when the latter is properly inserted on said cylindrical portion (101) of said bur (100), and where said at least one retaining component (300) is removably or non-removably constrained to said cylindrical portion (100), that is, it is part of said bur (100) and not of said stop element (200), as is the case in the prior art.

Said retaining element (300) is configured so as to create a negative allowance with said stop element (200) and in any case such as to preferably allow a relative rotation between the stop element (200) and bur (100).

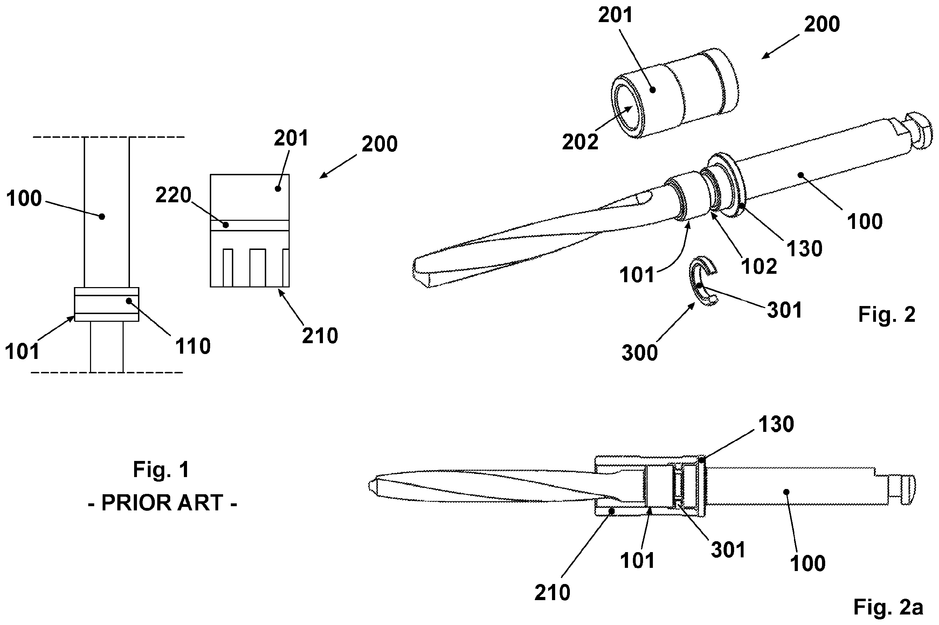

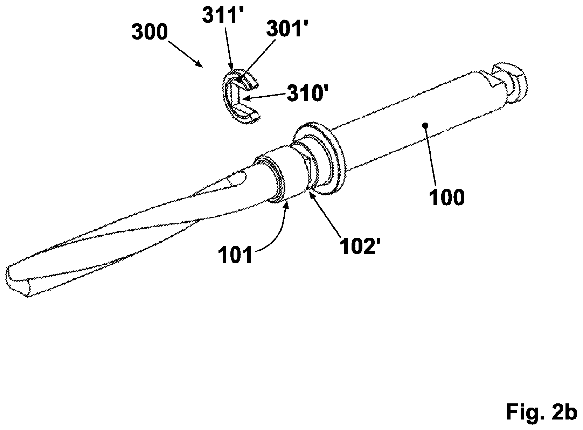

In the embodiment of Figures 2 and 2a, said at least one retaining component (300) comprises a whole or a part of a ring (301) inserted into a substantially circular groove (102) made on said cylindrical portion (101) of said bur (100), and wherein said ring (301) and said groove (102) are configured so that said ring (301) is protruding from said groove (102).

In particular, said ring (301) preferably has dimensions such as to be able to rotate within said groove (102) with respect to the cylindrical portion (101) of the bur (100) and/or such as to enable the rotation of said stop element (200) with respect to the bur (100).

Once said stop element (200) is inserted on said cylindrical portion (101) of said bur (100), said ring (301) creates the negative allowance necessary to retain said stop element (200) in the proper position.

The figures show how the bur (100), in any of its possible embodiments according to the present invention, may comprise a stop stroke element (130) against which said stop element (200), properly and completely inserted, must make contact.

Said ring (301) can be advantageously made, entirely or in part, of titanium, so as to be anodized with an identifying color.

In this embodiment, said stop element (200) can also be made of titanium and anodized with the same color as the ring (301) mounted on the bur (100) with corresponding dimensions.

Alternatively, said ring (301) may be made, entirely or in part, of steel, rubber, plastic or another suitable material, and may be colored with other technologies.

In the alternative embodiment of Figure 2b, said retaining component (300) comprises at least one whole ring or one partial ring (301') having a substantially circular outer surface (311') and a non-circular inner surface (310'), wherein said at least one ring (301') is inserted into a groove (102') made on said cylindrical portion (101) of said bur (100) and having a shape corresponding to said inner surface (310') of said ring (301'). Said ring (301') and said groove (102') are configured so that said ring (301') may be partially protruding from said groove (102').

In this way, said ring (301') does not rotate with respect to the bur (100) while a relative rotation between said stop element (200) and the bur (100) is allowed.

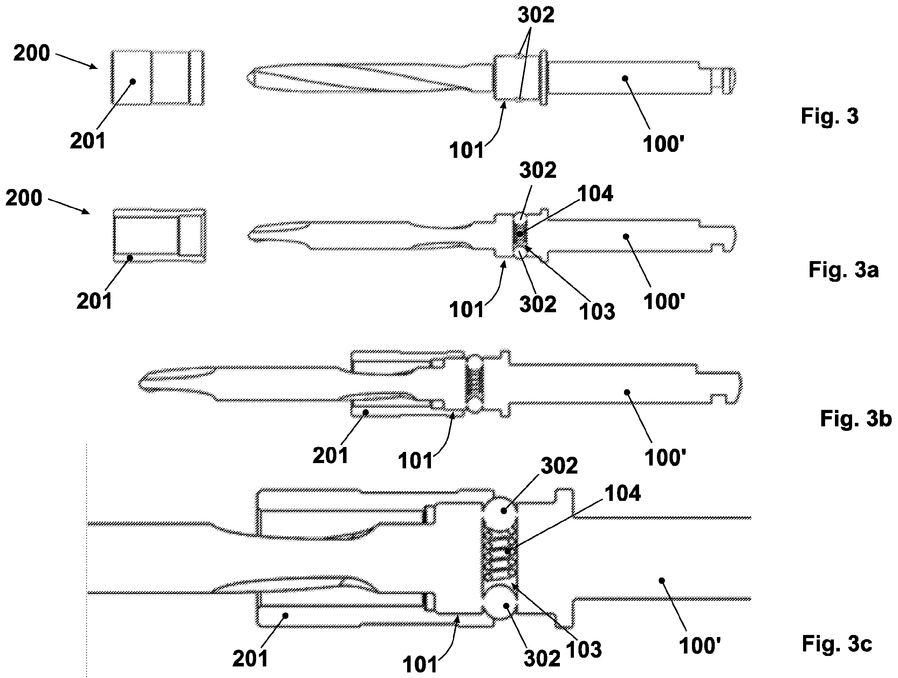

In the embodiment of Figures 3, said at least one retaining component (300) comprises at least one whole or partial ball (302) or at least one body inserted in a seat (103) located on said cylindrical portion (101) of said bur (100') by means of at least one elastic means (104), which constrains said ball (302) in a position at least partially protruding from said seat (103), counteracting the movement of the ball (302) towards the inside.

Once said stop element (200) is inserted on said cylindrical portion of said bur (100') and pushes the ball (302) towards the inside of the seat (103), said elastic means (104), in contrast, pushes the ball (302) towards the outside, so as to achieve the negative allowance.

Suitably, said bur (100') will comprise two or more of said balls (302) with relative seats (103) and elastic means (104), suitably arranged on said cylindrical portion (101) in a symmetrical manner with respect to the longitudinal axis of the bur (100').

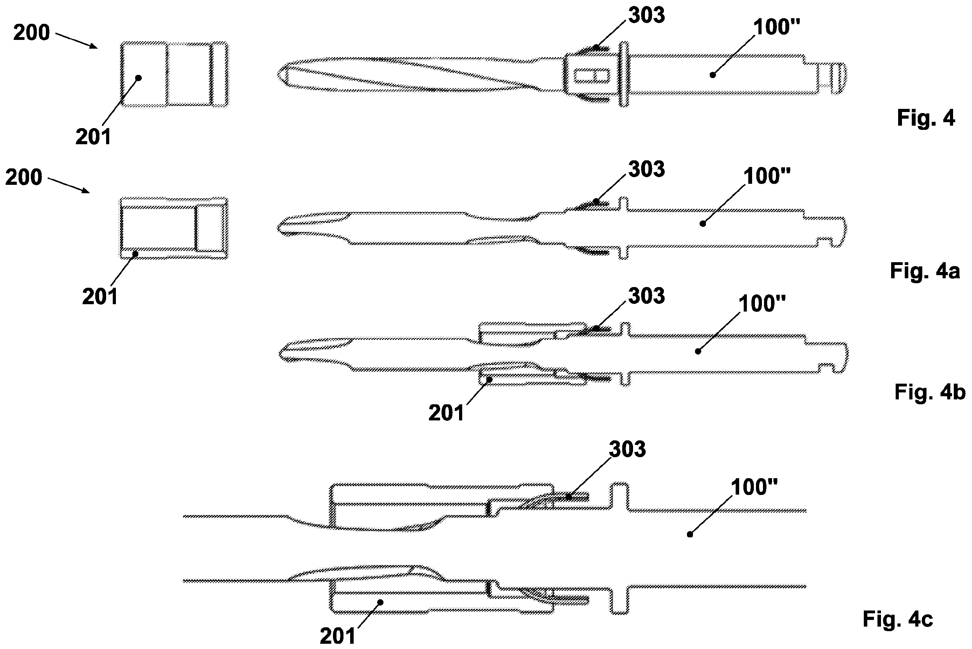

In the embodiment of Figures 4, said retaining component (300) comprises at least one elastically deformable element or projection (303) rising from said cylindrical portion (101) of the bur (100"). For example, said deformable element is a tooth oriented in a substantially longitudinal direction with respect to the bur's longitudinal axis.

Again, said bur (100") may comprise two or more of said elastically deformable elements or projections (303), distributed on said cylindrical portion (101) of the bur (100") symmetrically with respect to the longitudinal axis of the bur (100") itself.

Said stop element (200) may therefore not have the teeth of the prior art, but will comprise a cylindrical tubular body (201), with an axial hole (202) of a size suitable to allow the insertion of said cylindrical portion (101) of the bur (100) creating a negative allowance with said retaining component (300).

Therefore, with reference to the preceding description and the attached drawings the following claims are made.

## Claims

1. Component kit for dental implantology comprising a bur (100) for dental implantology, comprising a cylindrical portion (101) for the positioning of at least one stop element (200) used for adjusting the drilling depth, said bur (100) comprising at least one retaining component (300) suited to interfere with said stop element (200) when the latter is correctly inserted in said cylindrical portion (101) of said bur (100), and wherein said at least one retaining component (300) is constrained to said cylindrical portion (100) in a removable or non-removable manner, so that the elastic retaining function is carried out by the bur (100), wherein said component kit also comprises said stop element (200) in turn comprising a cylindrical tubular body (201) without tabs and provided with an axial hole (202) in a size suitable for the insertion of said cylindrical portion (101) of said bur (100) and for interfering with said at least one retaining component (300), **characterized in that** said at least one retaining component (300) comprises:
- at least one complete or partial ring (301, 301') inserted in a groove (102, 102') made on said cylindrical portion (101), and wherein said ring (301, 301') and said groove (102, 102') are configured in such a way that said ring (301, 301') partially projects from said groove (102, 102'),
- or
- at least one elastically deformable element or projection (303) which stands from said cylindrical portion (101) of the bur (100) and wherein said deformable element is a tooth oriented in a substantially longitudinal direction with respect to the bur's longitudinal axis.

2. Component kit according to claim 1, **characterized in that** said complete or partial ring (301) is inserted in a substantially circular groove (102) created on said cylindrical portion (101) of said bur (100).

3. Component kit according to claim 1, **characterized in that** said complete or partial ring (301') has an external surface (311') and an internal surface (310'), wherein the external surface (311') is substantially circular and the internal surface (310') is not circular, wherein said groove has a shape which corresponds to said internal surface (310') of said ring (301').

4. Component kit according to claim 2 or 3, **characterized in that** said ring (301) is completely or partially made of a material selected from among the following: titanium, steel, rubber, plastic.

5. Component kit according to claim 4, **characterized in that** said ring (301) is completely or partially coloured.

6. Component kit according to claim 5, **characterized in that** said ring (301) is made of titanium and coloured by means of an anodic process.

7. Component kit according to claim 5 or 6, **characterized in that** said stop element (200) is of the same colour as said at least one ring (301).

8. Component kit according to claim 2 or 3 , **characterized in that** the size of said ring (301, 301') is such that it can rotate inside said groove (102) with respect to the cylindrical portion (101) of the bur (100) and/or such as to allow said stop element (200) to rotate with respect to the bur (100).

## Patentansprüche

1. Komponentensatz für die dentale Implantologie, umfassend einen Bohrer (100) für die dentale Implantologie, der einen zylindrischen Abschnitt (101) zum Positionieren mindestens eines Stoppelements (200), das zum Anpassen der Bohrtiefe verwendet wird, umfasst, wobei der besagte Bohrer (100) mindestens eine Haltekomponente (300) umfasst, die dazu geeignet ist, mit dem besagten Stoppelement (200) in Eingriff zu treten, wenn dieses korrekt in den besagten zylindrischen Abschnitt (101) des besagten Bohrers (100) eingesetzt ist, und wobei die besagte mindestens eine Haltekomponente (300) am besagten zylindrischen Abschnitt (100) lösbar oder nicht lösbar befestigt ist, sodass die elastische Haltefunktion durch den Bohrer (100) ausgeführt wird, wobei der besagte Komponentensatz auch das besagte Stoppelement (200) umfasst, das wiederum einen zylindrischen Rohrkörper (201) ohne Laschen umfasst und mit einer Axialbohrung (202) in einer Größe versehen ist, die für das Einsetzen des besagten zylindrischen Abschnitts (101) des besagten Bohrers (100) und für das Eingreifen mit der besagten mindestens einen Haltekomponente (300) geeignet ist,
**dadurch gekennzeichnet, dass** die besagte mindestens eine Haltekomponente (300) Folgendes umfasst:
- mindestens einen vollständigen oder unvollständigen Ring (301, 301'), der in eine am besagten zylindrischen Abschnitt (101) hergestellte Nut (102, 102') eingesetzt ist, und wobei der besagte Ring (301, 301') und die besagte Nut (102, 102') so konfiguriert sind, dass der besagte Ring (301, 301') teilweise aus der besagten Nut (102, 102') herausragt, oder
- mindestens ein elastisch verformbares Element oder einen elastisch verformbaren Vorsprung (303), das bzw. der vom besagten zylindrischen Abschnitt (101) des Bohrers (100) absteht, und wobei das besagte verformbare Element ein Zahn ist, der in einer im Wesentlichen längs verlaufenden Richtung in Bezug auf die Längsachse des Bohrers ausgerichtet ist.

2. Komponentensatz nach Patentanspruch 1, **dadurch gekennzeichnet, dass** der besagte vollständige oder unvollständige Ring (301) in eine im Wesentlichen kreisförmige Nut (102) eingesetzt ist, die auf dem besagten zylindrischen Abschnitt (101) des besagten Bohrers (100) ausgebildet ist.

3. Komponentensatz nach Patentanspruch 1, **dadurch gekennzeichnet, dass** der besagte vollständige oder unvollständige Ring (301') eine Außenfläche (311') und eine Innenfläche (310') aufweist, wobei die Außenfläche (311') im Wesentlichen kreisförmig ist und die Innenfläche (310') nicht kreisförmig ist, wobei die besagte Nut eine Form aufweist, die der besagten Innenfläche (310') des besagten Rings (301') entspricht.

4. Komponentensatz nach Patentanspruch 2 oder 3, **dadurch gekennzeichnet, dass** der besagte Ring (301) ganz oder teilweise aus einem Material hergestellt ist, das aus den folgenden ausgewählt ist: Titan, Stahl, Gummi, Kunststoff.

5. Komponentensatz nach Patentanspruch 4, **dadurch gekennzeichnet, dass** der besagte Ring (301) ganz oder teilweise gefärbt ist.

6. Komponentensatz nach Patentanspruch 5, **dadurch gekennzeichnet, dass** der besagte Ring (301) aus Titan hergestellt und mittels eines anodischen Verfahrens gefärbt ist.

7. Komponentensatz nach Patentanspruch 5 oder 6, **dadurch gekennzeichnet, dass** das besagte Stoppelement (200) die gleiche Farbe wie der besagte mindestens eine Ring (301) aufweist.

8. Komponentensatz nach Patentanspruch 2 oder 3, **dadurch gekennzeichnet, dass** die Größe des besagten Rings (301, 301') derart ist, dass er sich innerhalb der besagten Nut (102) in Bezug auf den zylindrischen Abschnitt (101) des Bohrers (100) drehen kann und/oder derart, dass dem besagten Stoppelement (200) ermöglicht wird, sich in Bezug auf den Bohrer (100) zu drehen.

## Revendications

1. Kit de composants pour l'implantologie dentaire comprenant une fraise (100) pour l'implantologie dentaire, comportant une partie cylindrique (101) destinée au positionnement d'au moins un élément d'arrêt (200) servant à régler la profondeur de forage, ladite fraise (100) comportant au moins un élément de retenue (300) adapté pour venir en prise avec ledit élément d'arrêt (200) lorsque ce dernier est correctement inséré dans ladite partie cylindrique (101) de ladite fraise (100), et où ledit au moins un composant de retenue (300) est fixé à ladite partie cylindrique (100) de manière amovible ou non amovible, de sorte que la fonction de retenue élastique est assurée par la fraise (100), où ledit kit de composants comprend également ledit élément d'arrêt (200) comprenant à son tour un corps tubulaire cylindrique (201) sans ailettes et muni d'un trou axial (202) d'une taille adaptée à l'insertion de ladite partie cylindrique (101) de ladite fraise (100) et à l'interférence avec ledit au moins un composant de retenue (300),
**caractérisé en ce que** ledit au moins un élément de retenue (300) comprend :
- au moins un anneau complet ou partiel (301, 301') inséré dans une rainure (102, 102') pratiquée sur ladite partie cylindrique (101), et où ledit anneau (301, 301') et ladite rainure (102, 102') sont configurés de telle sorte que ledit anneau (301, 301') dépasse partiellement de ladite rainure (102, 102'), ou
- au moins un élément ou une saillie déformable élastiquement (303) qui s'étend à partir de ladite partie cylindrique (101) de la fraise (100) et où ledit élément déformable est une dent orientée dans une direction sensiblement longitudinale par rapport à l'axe longitudinal de la fraise.

2. Kit de composants selon la revendication 1, **caractérisé en ce que** ledit anneau complet ou partiel (301) est insérée dans une rainure sensiblement circulaire (102) créée sur ladite partie cylindrique (101) de ladite fraise (100).

3. Kit de composants selon la revendication 1, **caractérisé en ce que** ledit anneau complet ou partiel (301') présente une surface extérieure (311') et une surface intérieure (310'), où la surface extérieure (311') est sensiblement circulaire et la surface intérieure (310') n'est pas circulaire, où ladite rainure a une forme qui correspond à ladite surface intérieure (310') dudit anneau (301').

4. Kit de composants selon la revendication 2 ou 3, **caractérisé en ce que** ledit anneau (301) est complètement ou partiellement constitué d'un matériau choisi parmi les suivants : titane, acier, caoutchouc, plastique.

5. Kit de composants selon la revendication 4, **caractérisé en ce que** ledit anneau (301) est complètement ou partiellement coloré.

6. Kit de composants selon la revendication 5, **caractérisé en ce que** ledit anneau (301) est constitué de titane et coloré au moyen d'un procédé d'anodisation.

7. Kit de composants selon la revendication 5 ou 6, **caractérisé en ce que** ledit élément d'arrêt (200) est de la même couleur que ledit au moins un anneau (301).

8. Kit de composants selon la revendication 2 ou 3, **caractérisé en ce que** la taille dudit anneau (301, 301') est telle qu'il peut tourner à l'intérieur de ladite rainure (102) par rapport à la partie cylindrique (101) de la fraise (100) et/ou de manière à permettre audit élément d'arrêt (200) de tourner par rapport à la fraise (100).
